# EUROPEAN PATENT SPECIFICATION

(11) **EP 1 626 094 B1**
(45) Date of publication and mention of the grant of the patent: **30.07.2008**
(21) Application number: 05254945.8
(22) Date of filing: 08.08.2005
(51) Int. Cl.: C12Q 1/04

(54) **Method for cultivating microorganisms in the presence of fluoroquinolones and aminoglycosides**
Verfahren zur Kultivierung von Mikroorganismen in der Gegenwart von Fluoroquinolonen und Aminoglykosiden
Procédé pour la culture des micro-organismes en presence des aminoglycosides et fluoroquinolones

(30) Priority: 13.08.2004 US 601241 P
(43) Date of publication of application: 15.02.2006
(73) Proprietor: MILLIPORE CORPORATION, Billerica Massachusetts 01821 (US)
(72) Inventor: Souza, Kathleen, Westford, MA 01886 (US)
(74) Representative: Gambell, Derek

(56) References cited:
- WO-A-01/59157
- HAROLD EDDLEMAN: "Media for Bacteria Stock Cultures"[Online] 23 October 1999 (1999-10-23), pages 1-8, XP002356650 Retrieved from the Internet: URL:http://web.archive.org/web/19991023050 655/http://www.disknet.com/indiana_biolab/ b030.htm> [retrieved on 2005-11-29]
- BLANK J: "Enzymatic inactivation of residual gentamicin after membrane filtration." APPLIED AND ENVIRONMENTAL MICROBIOLOGY. JAN 1983, vol. 45, no. 1, January 1983 (1983-01), pages 319-320, XP002356651 ISSN: 0099-2240
- LÜLLMANN, H., MOHR, K., WEHLING, M.: "Pharmakologie und Toxikologie" 2003, GEORG THIEME VERLAG STUTTGART - NEW YORK , D-70469, STUTTGART , XP002356655 ISBN: 3133685155 * page 429, column 1 * * page 434, column 2 *
- ZIMELIS V M ET AL: "Activity of aminoglycoside antibiotics against Pseudomonas aeruginosa: specificity and site of calcium and magnesium antagonism" JOURNAL OF INFECTIOUS DISEASES 1973, vol. 127, no. 6, 1973, pages 663-669, XP008056443
- LOMAESTRO B M ET AL: "Quinolone-cation interactions: a review." DICP : THE ANNALS OF PHARMACOTHERAPY. NOV 1991, vol. 25, no. 11, November 1991 (1991-11), pages 1249-1258, XP008056513 ISSN: 1042-9611
- COOK M T: "Potato agar" THE OHIO NATURALIST, vol. X, no. 1, November 1909 (1909-11), pages 13-14,
- RUSSELL A D: "ANTIBIOTICS TODAY PART 3 RESISTANCE AND CROSS RESISTANCE COMBINED ANTIBIOTIC THERAPY AND STERILITY TESTING OF ANTIBIOTICS" PHARMACEUTICAL JOURNAL, vol. 201, no. 5468, 1968, pages 149-152, XP008076479
- RUSSELL A D; AHONKHAI I; ROGERS D T: "Microbiological applications of the inactivation of antibiotics and other antimicrobial agents." THE JOURNAL OF APPLIED BACTERIOLOGY, vol. 46, no. 2, April 1979 (1979-04), pages 207-245, XP008076490

## Description

The present invention relates to a method for recovering microorganisms in the presence of certain antibiotics. More particularly, it relates to a method for bioburden and sterility testing of such antibiotics, using a media which may also be used for environmental monitoring of microorganisms in the antibiotic manufacturing facilities.

### Background

Pharmaceuticals, ophthalmics and the like need to be sterile so as to not compromise or injure the user. They need to be tested to ensure that either any microbes that are present are at accepted low levels or that no microbes exist at all before the product is released.

For most products, powder or liquid, the process is as follows:

The sample of the liquid (or of a powder dissolved into a liquid) is filtered through a microporous filter having a pore size small enough to capture any microorganisms on its surface.

The filter is then either placed on a growth medium such as an agar plate or in a medium such as a broth or a growth medium is applied to filter or an absorptive pad below filter and incubated, either at room temperature or at elevated temperatures (98° F or so) for a period of time to allow any microorganisms to grow to a size sufficient to be enumerated and if desired identified.

The test for enumeration and identification can be visual (simply counting the number of colonies that form) or alternatively it may be done through the use of various agents to detect the presence of the microbes and to provide a signal (bio- or chemi- luminescent, radiologic, colorimetfic and the like) that can be seen by the eye or through instrumentation.

One type of product that is difficult to test is antibiotics selected from fluoroquinolones, and aminoglycosides.

Antibiotics are designed to kill or inhibit bacteria and other microorganisms. The current test uses a series of washes to attempt to remove all traces of the antibiotic from the filter so that microbe growth is not inhibited. This is a time consuming, costly procedure and it doesn't always work. What is needed is a better methodology for the bioburden, sterility and environmental testing of antibiotics.

We are aware of the following art.

HAROLD EDDLEMAN: "Media for Bacteria Stock Cultures" 23 October 1999 (1999-10-23), pages 1-8, which describes in particular a medium for many myxobacteria, comprising magnesium sulphate, agar, casitone and distilled water.

BLANK J: "Enzymatic inactivation of residual gentamicin after membrane filtration." APPLIED AND ENVIRONMENTAL MICROBIOLOGY. JAN 1983, vol. 45, no. 1, January 1983 (1983-01), pages 319-320, which describes the inactivation of residual gentamicin on a membrane by modification with two different enzymes.

LÜLLMANN, H., MOHR, K., WEHLING, M.: "Pharmakologie und Toxikologie" 2003, GEORG THIEME VERLAG STUTTGART - NEW YORK , D-70469, STUTTGART, discloses that tetracyclines variably form complexes with divalent cations, whereby the resulting complexes no longer function as antibiotics.

ZIMELIS V M ET AL: "Activity of aminoglycoside antibiotics against Pseudomonas aeruginosa: specificity and site of calcium and magnesium antagonism" JOURNAL OF INFECTIOUS DISEASES 1973, vol. 127, no. 6, 1973, pages 663-669, discloses that adding divalent cations to a culture medium alters the antimicrobial action against *Pseudomonas aeruginosa.*

LOMAESTRO B M ET AL: "Quinolone-cation interactions: a review." DICP : THE ANNALS OF PHARMACOTHERAPY. NOV 1991, vol. 25, no. 11, November 1991 (1991-11), pages 1249-1258, discloses that *in vivo* a quinolone class of antibiotics can form chelates and are inactivated by cations such as magnesium, aluminium, iron, zinc and calcium.

RUSSELL A D: "ANTIBIOTICS TODAY PART 3 RESISTANCE AND CROSS RESISTANCE COMBINED ANTIBIOTIC THERAPY AND STERILITY TESTING OF ANTIBIOTICS" PHARMACEUTICAL JOURNAL, vol. 201, no. 5468, 1968, pages 149-152, describes ways of sterility testing of antibiotics. Strategies involve the inactivation of the antibiotics by inactivating agents and washing off residual antibiotics. On p.151 : col2, lines 4 to 12 it is described that Fe⁺⁺ and Mg⁺⁺ revive tetracylcine-treated bacteria, and a method in which an appropriate metallic salt is included in media to which an antibiotic is added is suggested for sterility testing of certain antibiotics.

RUSSELL A D; AHONKHAII; ROGERS D T: "Microbiological applications of the inactivation of antibiotics and other antimicrobial agents." THE JOURNAL OF APPLIED BACTERIOLOGY, vol. 46, no. 2, April 1979 (1979-04), pages 207--245, considers the possible methods of inactivating many types of antibiotics and other antimicrobial agents and the situations in which such inactivation is necessary.

Finally, we are aware of international patent publication WO 01/59157 (Millipore Corporation) which describes a test for the detection, enumeration and identification of microorganisms in a fluid sample is taught. The test uses a filter through which a fluid sample is passed and onto which organisms within the fluid are deposited. It is then incubated on a growth medium and thereafter subjected to a presence absence test for organisms using an ATP/bioluminescence test. The same sample and filter is then subjected to a PNA probe assay for targeted organisms. Optionally, the locations of the organisms detected by each test can be compared with each other to determine the possibility of false positives and their elimination from the test results.

### SUMMARY OF THE INVENTION

According to the present invention, there is provided a process for determining the existence of microbes in the presence of an antibiotic, wherein the antibiotic is selected from fluoroquinolones and aminoglycosides, comprising the steps of: providing a sample containing the antibiotic to be tested, a holder and a growth medium within the holder; placing the sample in contact with the growth media and incubating the media for a pre-selected time at a pre-selected temperature; and viewing the media to determine the presence of any microorganisms. The invention is characterised in that the medium contains one or more cation constituents selected from divalent and trivalent cations of magnesium, calcium, aluminium and iron and said cations range in concentration from 0.1 M to 0.5M.

The invention is particularly appicable where the antibiotic is selected from fluoroquinolones and is particularly effective where the microbe is selected from *S. aureus* and *P. aeruginosa.*

Thus, the present invention provides a method of using a specified medium for the bioburden or sterility testing of fluoroquinolones and aminoglycosides or for testing the fluoroquinolones and aminoglycosides manufacturing environment. The medium contains one or more divalent or trivalent cation constituents of magnesium, calcium, aluminium and iron in concentration range from 0.1-0.5M that allow for microorganism growth even in the presence of residual fluoroquinolones and aminoglycosides.

The method preferably comprises filtering the fluoroquinolones and aminoglycosides sample through a filter having a pore size small enough to capture the suspected microorganisms and then incubating that filter on or in a growth medium that contains one or more divalent or trivalent cation constituents of magnesium, calcium, aluminium and iron in a concentration range from 0,1-0,5M. The incubated filter or medium filter or medium is then viewed or tested to determine the presence and if so, number and desirably, type of microorganisms present.

An alternative for bioburden or sterility testing is by directly inoculating or plating the sample comprising fluoroquinolones or aminoglycosides in or on the medium that contains one or more divalent or trivalent cation constituents of magnesium, calcium, aluminium and iron in a concentration of 0.1M - 0.5M further method to pull air onto a filter or directly onto or into a medium that contains one or more divalent or trivalent cations of magnesium, calcium, aluminium and iron in a concentration of 0.1M - 0.5M.

### Detailed Description

The present invention makes use of a growth media that contains one or more divalent or trivalent cation constituents of magnesium, calcium, aluminium and iron in a concentration of 0.1M - 0.5M within them. The presence of the one or more divalent or trivalent cation constituents them. The presence of the one or more divalent or trivalent cation constituents allows microorganisms to grow even in the presence of residual fluoroquinolones and aminoglycosides. It is useful for bioburden or sterility testing of the fluoroquinolones and aminoglycosides where the presence of residual antibiotics inhibits microorganism growth and provides a false negative result. It is also useful for environmental monitoring of air in an antibiotic manufacturing environment.

Any divalent or trivalent cation constituent may be used in the present invention. Preferred examples include but are not limited to magnesium, preferably in the form of magnesium sulfate and magnesium chloride: calcium preferably in the form of calcium chloride or calcium citrate; aluminum sulfate; and iron (ferrous) sulfate.

The amount of divalent or trivalent cation present in the media should be sufficient to overcome the antibiotic inhibition so as to allow any microorganisms present to grow. Generally It should be present in an amount from 0.1M to 0.5M preferably from 0.2 M to 0.4M and more preferably about 0.3M.

Media are typically in the form of a gel, such as agar-based media or in the form of a liquid, such as broths. The media typically used in these tests are Soybean Casein Digest Broth or Agar (SCDB or SCDA), Fluid Thioglycollate Medium (FTM) and Sabouraud Dextrose Agar (SDA). Other media that are useful include but not limited to Mueller Hinton Broth or Agar, Nutrient Broth or Agar (agar may be in special cassettes or in a standard agar Petri plate).

A typical method for testing for bioburden levels or sterility of the antibiotics is to filter a sample of the antibiotic through a filter having a pore size small enough to trap any microorganisms and then incubate that filter in the presence of a growth medium to allow the microorganisms to grow to a size suitable for detection.

Such filters typically have a pore size of 0.45 micrometres (microns) or less; in some instances a pore size from about 0.1 micrometers up to 1.2 micrometers is preferred. The filters can be formed of any suitable material commonly used for such applications including but not limited to cellulose based filters such as regenerated cellulose, mixed cellulose esters, cellulose acetate, cellulose nitrate, nitro cellulose and the like, PVDF, nylons, polycarbonates and polysulfones such as polysulfone, polyethersulfone, polyarylsulfone and polyphenylsulfone.

Such filters are commercially available from a number of suppliers. Suitable filters include S-Pak™ mixed cellulose ester filters and Durapore® PVDF filters available from Millipore Corporation of Billerica, Massachusetts.

Holders for the filters may simply be a stainless device such as a funnel or it may be a disposable, presterilized filter containing device such as a Steritest™ device, a MicropreSure™ device, Sterisure® device, a Milliflex® filter unit or a Microfil® S device, all available from Millipore Corporation of Billerica, Massachusetts.

Especially for sterility testing, the use of an enclosed test, such as a Steritest™ device, allows one to conduct the entire test (sampling, filtration, media addition and incubation) in a closed system. This design dramatically reduces the risks of adventitious contamination and subsequent false positives.

A typical method for testing for environmental levels of microbes in a facility is to filter a sample of air through a device having a media cassette on to which the microbes can be placed and retained and then incubate on that media to allow the microorganisms to grow to a size suitable for detection. One such system is known as the M Air T® system available from Millipore Corporation of Billerica, Massachusetts. Also see US 6,094,997 and 6,240,768. Other methods include simply leaving opened Petri dishes filled with a selected medium out in the environment to be studied and allowing falling microbes to collect on the medium's surface. The dishes are then incubated and viewed. Other methods can be and are used by those of ordinary skill in the art.

Generally, the sample after application to the media is incubated for a period of time to enable some growth of the captured microorganisms so that they can be easily detected. For traditional methods, this time can range from a minimum of 3 days for an air monitoring or bioburden sample to 14 days for the sterility test. Generally it is between about 3-14 days, more generally between about 7 and 14 days. The sample may be incubated at room temperature (around 20° C) up to higher temperatures such as around 54°C. Typical temperature range is 20 to 35° C.

The test for enumeration and identification can be visual (simply counting the number of colonies that form) either with the naked eye or through a microscope or other magnifying device. Altematively, it may be more complex and use various agents to detect the presence of various microbe constituents such as such as probes for DNA or RNA, agents for ATP; bioluminescence and other such well know chemical/biochemical agents to indicate the presence of these constituents and/or instruments to detect these agents to indicate the existence of and type of organisms present. For example, one well-known system incubates the microbes, lyses them and then uses reagents to detect the ATP within them. The presence of the ATP is visualized by a bioluminescent reaction of luciferine and luciferase. One such system is sold as the Microstar® system available from Millipore Corporation of Billerica, Massachusetts. Other systems based on chromatographic indicators, fluorescent indicators, and the like are also known in the art.

### Example 1 (direct inoculation)

Test tubes containing SCDB media in the presence or absence of magnesium cation or ciprofloxacin antibiotic were inoculated with 200 colony forming units (cfu) of *S. aureus* (ATCC 6538). Sample A was used with a ciprofloxacin sample (100 µg/mL) and contained no divalent cation constituent. B was used with ciprofloxacin (100 µg/mL) and contained 0.5 M magnesium cation. C was used with a control and contained 0.5M of the same divalent cation of B. D was used with a control and contained no divalent cation.

The results are shown in Table 1:

**Table 1**

| **Antibiotic** | **Microorganism** | **Divalent cation added to broth** | **Result** |
|---|---|---|---|
| Ciprofloxacin* | *S. aureus* | None | No growth |
| Ciprofloxacin | *S. aureus* | 0.5M | Growth |
| None* | *S. aureus* | 0.5M | Growth |
| None* | *S. aureus* | None | Growth |

| | | | |
|---|---|---|---|
| * comparative example | | | |

### Example 2 (filtered)

A sample of ofloxacin antibiotic (10ml of at 4mg/mL) was filtered through two of four Milliflex® funnels containing a Durapore® membrane. Each membrane filter was rinsed with six, 100 mL rinses of USP Fluid A, the last rinse containing approximately 20 to 60 cfu of *E*. *coli* (ATCC 8739). A control of *E. coli* in USP Fluid A was also filtered through a Milliflex® funnel. All membranes were then placed on growth media (Soybean Casein Digest Agar) as samples A, B, C, and D. A was used with an ofloxacin sample and contained no divalent cation constituent. B was used with ofloxacin and contained 0.5 M divalent cation (magnesium). C was used with a control and contained 0.5% divalent cation of B. D was used with a control and contained no divalent cation.

The results are shown in Table 2:

**Table 2**

| **Antibiotic** | **Microorganism** | **Divalent cation added to agar** | **Result** |
|---|---|---|---|
| Ofloxacin* | *E. coli* | None | No growth |
| Ofloxacin | *E. coli* | 0.5M | Growth |
| None* | *E. coli* | 0.5M | Growth |
| None* | *E. coli* | None | Growth |

| | | | |
|---|---|---|---|
| * comparative example | | | |

As can be seen from the examples in the presence of the antibiotics, microorganism growth was inhibited unless the media contained a divalent cation. This allows for bioburden or sterility testing of antibiotics to occur while limiting or eliminating the potential for false negatives.

### Example 3 (sterility testing)

A sample of ofloxacin antibiotic (20 ml of at 40 mg/mL) was filtered through two of four paired Steritest™ canister sets each containing a Durapore® membrane. Each membrane filter was rinsed with three, 100 mL rinses of USP Fluid A, the last rinse containing approximately 30 cfu of *B. subtilis* (ATCC 6633). A control of *B. subtilis* in USP Fluid A was also filtered through a Steritest device. Growth media (Soybean Casein Digest Agar) A, B, C, and D was then added to each canister.

The results are shown in Table 3:

**Table 3**

| **Antibiotic** | **Microorganism** | **Divalent cation added to broth** | **Result** |
|---|---|---|---|
| Ofloxacin* | *B. subtilis* | None | No growth |
| Ofloxacin | *B. subtilis* | 0.5M | Growth |
| None* | *B. subtilis* | 0.5M | Growth |
| None* | *B. subtilis* | None | Growth |

| | | | |
|---|---|---|---|
| * comparative example | | | |

Similar results were demonstrated with ofloxacin with SCDB containing a divalent cation at concentrations of 0.1, 0.2, 0.3 and 0.4 M magnesium cation. In addition, similar results were demonstrated with two additional fluoroquinolones; moxifloxacin and ciprofloxacin with SCDB containing divalent cation (magnesium) at 0.3M concentration.

### Example 4 (air monitoring testing)

One method of monitoring air in an antibiotic manufacturing plant is to impact air onto an agar surface collecting both microorganisms and antibiotic on the agar surface. This test was simulated by spreading microorganisms over an agar surface containing varying amounts of magnesium cation and then placing disks containing antibiotics onto the agar surface. The zone of inhibition around each disk was measured to give an indication of the ability of the medium to neutralize the effect of the antibiotic (method followed is similar to the susceptibility disc test used in clinical microbiology).

S. *aureus* (ATCC 6538), *P. aeruginosa* (ATCC 9027) or *E. coli* (ATCC 25922) were spread on the agar surface of SCDA containing 0.1M, 0.2M, 0.3M, 0.4M or 0.5M magnesium cation. Discs impregnated with a known amount of antibiotic were then placed onto the bacteria on the agar plate. Plates were incubated and the zone of inhibition of bacterial growth was measured. A decrease in this zone of inhibition of bacterial growth demonstrates the protective effect of the medium containing cation. Results for three antibiotics, ofloxacin (a fluoroquinolone), streptomycin (an aminoglycoside) and, for the sake of comparison, doxycycline (a tetracycline), are shown in Table 4.

**Table 4**

| | | Zone of Inhibition in mm | | | | | |
|---|---|---|---|---|---|---|---|
| **Antibiotic** | **Microorganism** | | | | | | |
| | | 0.0M cation | 0.1M cation | 0.2M cation | 0.3M cation | 0.4M cation | 0.5M cation |
| Ofloxacin (5ug) | *S. aureus* | 23 | 11 | 8 | 6 | 7 | 6 |
| Ofloxacin (5ug) | *P. aeruginosa* | 20 | 6 | 6 | 6 | 6 | 6 |
| Ofloxacin (5ug) | *E. coli* | 26 | 13 | 13 | 13 | 12 | 12 |
| | | | | | | | |
| Streptomycin (10ug) | *S. aureus* | 17 | 12 | Np | 7 | Np | 8 |
| Streptomycin (10ug) | *P. aeruginosa* | 11 | 6 | Np | 6 | Np | 6 |
| Streptomycin (10ug) | *E. coli* | 14 | 12 | Np | 9 | Np | 6 |
| | | | | | | | |
| Doxycycline (30ug)* | *S. aureus* | 25 | 13 | Np | 11 | Np | 13 |
| Doxycycline (30ug)* | *P. aeruginosa* | 6 | 6 | Np | 6 | Np | 6 |
| Doxycycline (30ug)* | *E. coli* | 18 | 6 | Np | 6 | Np | 6 |

| | | | | | | | |
|---|---|---|---|---|---|---|---|
| Np: not performed * comparative example | | | | | | | |

Similar results were observed with eight other fluoroquinolone agents, ciprofloxacin, moxifloxacin, enoxocin, enrofloxacin, levofloxacin, lemofloxacin, norfloxacin and sparfloxacin.

## Claims

1. A process for determining the existence of microbes in the presence of an antibiotic, wherein the antibiotic is selected from fluoroquinolones and aminoglycosides, comprising the steps of:
providing a sample containing the antibiotic to be tested, a holder and a growth medium within the holder;
placing the sample in contact with the growth media and incubating the media for a pre-selected time at a pre-selected temperature; and
viewing the media to determine the presence of any microorganisms,
**characterised in that** the medium contains one or more cation constituents selected from divalent and trivalent cations of magnesium, calcium, aluminium and iron and said cations range in concentration from 0.1M to 0.5M.

2. The process of claim 1 further comprising
a) providing a filter, filtering the sample through the filter and placing the filter onto the growth media before incubation, or
b) providing a filter, filtering the sample through the filter and placing the filter onto the growth media before incubation and wherein the filter has a pore size of from 0.1 micrometre to 1.2 micrometre and is formed of a material selected from regenerated cellulose, mixed cellulose esters, cellulose acetate, cellulose nitrate, nitrocellulose, PVDF, nylons, polycarbonates, polysulfones, polyethersulfones, polyarylsulfones and polyphenylsulfones.

3. The process of claim 1 wherein
a) the sample is selected from a liquid and a powder dissolved in a liquid, or
b) the sample is air from a manufacturing area of antibiotic manufacturing, or
c) the filter/media are incubated for a period of time from about 0 to 7 days at a temperature from 20°C to 54°C, or
d) the filter/media are incubated for a period of time from 7 to 14 days at a temperature from 20°C to 54°C, or
e) the viewing is selected from visual counting of the colony forming units or bio- or chemi- luminescent detection of the presence of a microbe constituent, or
f) the viewing is by the detection of an agent used to indicate the presence of a microbe constituent.

4. The process of claim 1 wherein the one or more cations constituents are in a range of from 0.2M to 0.4M.

5. The process of claim 1, wherein the medium is in the form of a gel or liquid.

6. The process of claim 1, wherein the antibiotic is selected from fluoroquinolones.

7. The process of claim 1, wherein the mibrobes are selected from S. aureus and P. *aeruginosa.*

## Patentansprüche

1. Verfahren zur Bestimmung der Existenz von Mikroben in der Gegenwart eines Antibiotikums, wobei das Antibiotikum ausgewählt wird aus Fluorchinolonen und Aminoglykosiden, aufweisend die Schritte:
Zur Verfügung stellen einer Probe, enthaltend das zu testende Antibiotikum, einen Halter und ein Wachstumsmedium in dem Halter,
Platzieren der Probe in Kontakt mit dem Wachstumsmedium und Inkubieren des Mediums für eine vorgewählte Zeit bei einer vorgewählten Temperatur und
Betrachten des Mediums, um die Gegenwart von irgendwelchen Mikroorganismen zu bestimmen,
**dadurch gekennzeichnet, dass** das Medium eine oder mehrere Kationenbestandteile enthält, ausgewählt aus divalenten und trivalenten Kationen von Magnesium, Kalzium, Aluminium und Eisen, und dass die Kationen in einer Konzentration von 0,1 M bis 0,5 M rangieren.

2. Verfahren nach Anspruch 1, weiterhin aufweisend
a) zur Verfügung stellen eines Filters, Filtrieren der Probe durch den Filter und Platzieren des Filters auf dem Wachstumsmedium vor der Inkubation oder
b) zur Verfügung stellen eines Filters, Filtrieren der Probe durch den Filter und Platzieren des Filters auf dem Wachstumsmedium vor der Inkubation und wobei der Filter eine Porengröße von 0,1 Mikrometer bis 1,2 Mikrometer hat und gebildet wird aus einem Material, ausgewählt aus regenerierter Zellulose, gemischten Zelluloseestern, Zelluloseacetat, Zellulosenitrat, Nitrozellulose, PVDF, Nylons, Polycarbonaten, Polysulfonen, Polyethersulfonen, Polyarylsulfonen und Polyphenylsulfonen.

3. Verfahren nach Anspruch 1, wobei
a) die Probe ausgewählt wird aus einer Flüssigkeit und einem Pulver, gelöst in einer Flüssigkeit, oder
b) die Probe Luft aus einem Herstellungsbereich zur Antibiotika-Herstellung ist, oder
c) der Filter/das Medium für einen Zeitraum von etwa 0 bis 7 Tagen bei einer Temperatur von 20°C bis 54°C inkubiert werden, oder
d) der Filter/das Medium für einen Zeitraum von 7 bis 14 Tagen bei einer Temperatur von 20°C bis 54°C inkubiert werden, oder
e) das Betrachten ausgewählt wird aus visuellem Zählen der Kolonie bildenden Einheiten oder Bio- oder Chemilumineszenz-Detektion der Gegenwart eines Mikrobenbestandteils, oder
f) das Betrachten durch Detektion eines Mittels vorgenommen wird, welches dazu verwendet wird, um die Gegenwart eines Mikrobenbestandteils anzuzeigen.

4. Verfahren nach Anspruch 1, wobei die ein oder mehrere Kationenbestandteile in einem Bereich von 0,2 M bis 0,4 M sind.

5. Verfahren nach Anspruch 1, wobei das Medium in der Form eines Gels oder einer Flüssigkeit ist.

6. Verfahren nach Anspruch 1, wobei das Antibiotikum ausgewählt wird aus Fluorchinolonen.

7. Verfahren nach Anspruch 1, wobei die Mikroben ausgewählt werden aus S. aureus und P. aeruginosa.

## Revendications

1. Procédé pour déterminer l'existence de microbes en présence d'un antibiotique, dans lequel l'antibiotique est sélectionné parmi des fluoroquinolones et des aminoglycosides, comportant les étapes consistant à :
fournir un échantillon contenant l'antibiotique à tester, un support et un milieu de croissance dans le support,
mettre l'échantillon en contact avec le milieu de croissance et laisser incuber le support pendant un temps présélectionné à une température présélectionnée, et
examiner le milieu pour déterminer la présence de micro-organismes quelconques,
**caractérisé en ce que** le milieu contient un ou plusieurs constituants cationiques sélectionnés à partir de cations divalents et trivalents de magnésium, calcium, aluminium et fer et lesdits cations étant dans une plage de concentration de 0,1 M à 0,5 M.

2. Procédé selon la revendication 1, consistant de plus :
a) à fournir un filtre, filtrer l'échantillon à travers le filtre et mettre le filtre sur le milieu de croissance avant incubation, ou
b) fournir un filtre, filtrer l'échantillon à travers le filtre et mettre le filtre sur le milieu de croissance avant incubation et dans lequel le filtre a une taille de pore d'environ 0,1 micron à environ 1,2 micron et est formé d'un matériau sélectionné parmi de la cellulose régénérée, des esters de cellulose mélangés, de l'acétate de cellulose, du nitrate de cellulose, de la nitrocellulose, PVDF, des Nylons, des polycarbonates, des polysulfones, des polyéthersulfones, des polyarylsulfones et des polyphénylsulfones.

3. Procédé selon la revendication 1, dans lequel :
a) l'échantillon est sélectionné à partir d'un liquide et d'une poudre dissoute dans un liquide, ou
b) l'échantillon est de l'air provenant d'une zone industrielle de fabrication d'antibiotiques, ou
c) le filtre/milieu sont mis à incuber pendant une période de temps d'environ 0 à 7 jours à une température de 20 °C à 54°, ou
d) le filtre/milieu sont mis à incuber pendant une période de temps de 7 à 14 jours à une température de 20 °C à 54 °C, ou
e) l'examen est sélectionné parmi une numération visuelle des unités formant une colonie ou une détection bio- ou chimio-luminescente de la présence d'un constituant microbien, ou
f) l'examen est fait par la détection d'un agent utilisé pour indiquer la présence d'un constituant microbien.

4. Procédé selon la revendication 1, dans lequel un ou plusieurs constituants cationiques sont dans une plage allant de 0,2 M à 0,4 M.

5. Procédé selon la revendication 1, dans lequel le milieu est sous la forme d'un gel ou d'un liquide.

6. Procédé selon la revendication 1, dans lequel l'antibiotique est sélectionné à partir de fluoroquinolones.

7. Procédé selon la revendication 1, dans lequel les microbes sont sélectionnés parmi *S. aureus* et *P. aeruginosa.*
